# EUROPEAN PATENT SPECIFICATION

(11) **EP 3 720 496 B1**
(45) Date of publication and mention of the grant of the patent: **05.02.2025**
(21) Application number: 18821993.5
(22) Date of filing: 07.12.2018
(51) Int. Cl.: A61K 47/02, A61K 9/00, A61K 9/14, A61K 9/20, A61K 31/465, A61P 25/34

(54) **SOLID ORAL NICOTINE FORMULATION**
FESTE ORALE NIKOTINFORMULIERUNG
FORMULATION DE NICOTINE ORALE SOLIDE

(30) Priority: 08.12.2017 DK PA201770926
(43) Date of publication of application: 14.10.2020
(60) Divisional application: 24220229.9
(73) Proprietor: Fertin Pharma A/S, 7100 Vejle (DK)
(72) Inventor: NIELSEN, Bruno Provstgaard, 7100 Vejle (DK); NIELSEN, Kent Albin, 7100 Vejle (DK)
(74) Representative: Patentgruppen A/S
(86) International application number: PCT/DK2018/050336
(87) International publication number: WO 2019/110073

(56) References cited:
- EP-A1- 2 177 213
- WO-A1-02/085119
- WO-A1-2007/133140
- WO-A1-2013/091631
- US-A1- 2002 002 189
- US-A1- 2013 289 079
- "Crospovidone ED - Rowe Raymond C; Sheskey Paul J; Weller Paul J", 26 November 2002, HANDBOOK OF PHARMACEUTICAL EXCIPIENTS, PHARMACEUTICAL PRESS, LONDON, UK, PAGE(S) 184, ISBN: 978-0-85369-537-0, XP002719370
- ROWE RAYMOND C ET AL: "Handbook of pharmaceutical excipients, Mannitol", 1 January 2006, HANDBOOK OF PHARMACEUTICAL EXCIPIENTS, PHARMACEUTICAL PRESS [U.A.], LONDON [U.A.], PAGE(S) 449 - 453, ISBN: 978-1-58212-058-4, XP002537758

## Description

### FIELD OF THE INVENTION

The present invention relates to the field of solid oral nicotine formulations.

### BACKGROUND OF THE INVENTION

Nicotine-releasing formulations applied for the purpose of providing a release of nicotine in a user's mouth over a certain period are well-known. Much effort has in prior art been put into emulating the nicotine release and oral perception of a cigarette when it is smoked by a user, which means that release profiles from nicotine formulations have been thoroughly investigated in prior art. Patent document US 2002/0002189 discloses a nicotine therapy method and an oral carrier for assuaging tobaccoaddiction.

It is however an established fact that the one-to-one smoking-emulation is yet to be achieved with other means than a cigarette.

### SUMMARY

The invention relates to an orally disintegrating nicotine tablet for fast onset nicotine craving relief comprising a pressed powder formulation, the formulation comprising nicotine and a pH regulating agent, wherein the orally disintegrating nicotine tablet is designed to release the content of nicotine within a period of 60 seconds in contact with oral saliva and the orally disintegrating nicotine tablet is designed to release the content of pH regulating agent within a period of 60 seconds in contact with oral saliva, wherein the orally disintegrating nicotine tablet comprises at least one polyol, wherein the polyol comprises more than 40% by weight of the orally disintegrating nicotine tablet, wherein the at least one polyol is selected from the list consisting of sorbitol, erythritol, xylitol, maltitol, mannitol, lactitol, and isomalt, wherein the orally disintegrating nicotine tablet comprises nicotine in an amount of at least 0.5 mg, wherein the nicotine is provided as free base nicotine or nicotine salt, and wherein the pH regulating agent is an alkaline buffering agent, and wherein the nicotine is without carrier.

One significant advantage of the invention may be that a nicotine craving relief may be provided in a manner that is both fast and pleasant for the user, thus facilitating a more effective relief of nicotine craving since the critical time span between the use of the formulation and the relief, in which a user may otherwise be tempted to use tobacco products to satisfy the craving, is minimized, and since the otherwise common and highly unpleasant burning sensation may be avoided or minimized.

One advantage of the invention may be that fast onset nicotine craving relief may be obtained when using the solid oral nicotine formulation of the invention, while at the same time avoiding or at least minimizing the burning sensation, which is otherwise typically associated with nicotine products, especially fast release nicotine products. The fast onset nicotine craving relief is obtained by facilitating release the content of nicotine within 60 seconds in contact with oral saliva, providing a high nicotine concentration in saliva, while at the same time facilitating release the content of pH regulating agent within a period of 60 seconds in contact with oral saliva, thereby providing optimized conditions for fast nicotine uptake through the oral mucosa.

Contrary to expectations, experiments have shown that the permeability of nicotine across the buccal mucosa decreases relatively little when increasing the concentration of nicotine. For example, experiments have shown that an increase in the concentration of nicotine from 100 microgram / mL to 14,000 microgram / mL results in a decrease of about a factor of two. This is highly surprising and is utilized by aiming for concentrations of nicotine in the oral cavity, which are much higher than previously seen or desired. The present delivery vehicle thus benefits and aims for very high nicotine content in the oral cavity, thereby increasing the nicotine uptake. Furthermore, it has been realized that the effect of nicotine concentrations is thus at least comparable to the effect of pH regulation in the oral cavity. This is contrary to any expectations.

The above has been accomplished to the surprise of the inventors, as typical conventional products and conventional wisdom seek to delay the disintegration and delay the dissolution of nicotine.

Thus, an advantage of the present invention may be that solid oral nicotine formulation provides a highly efficient fast onset nicotine craving relief which is pleasant for the user, since the burning sensation is decreased or avoided.

In an embodiment of the invention the formulation is designed to release the content of nicotine within a period of between 5 and 60 seconds in contact with oral saliva, such as between 10 and 60 seconds in contact with oral saliva.

According to the claims, the formulation comprises nicotine in an amount of at least 0.5 mg.

According to an embodiment of the invention, the formulation comprises nicotine in an amount of between 0.5 mg and 10.0 mg, such as between 0.5 mg and 4.0 mg.

The amount of nicotine content is generally given in amount per dosage unless otherwise specified. If the dosage is in the form of a tablet, the amount will refer to the complete tablet.

Thus, according to an embodiment of the invention, the formulation comprises nicotine in an amount of at least 0.5 mg per dosage.

According to the claims, the formulation is designed to release the content of nicotine within a period of 60 seconds in contact with oral saliva.

In an embodiment of the invention, the formulation is designed to release the content of nicotine within a period of between 5 and 60 seconds in contact with oral saliva, such as between 10 and 40 seconds in contact with oral saliva.

According to an embodiment of the invention the formulation is designed to release the content of nicotine within a period of 30 seconds in contact with oral saliva. According to an embodiment of the invention the formulation is designed to release the content of nicotine within a period of between 5 and 30 seconds in contact with oral saliva, such as between 10 and 30 seconds in contact with oral saliva. According to an embodiment of the invention the formulation is designed to release the content of nicotine within a period of between 10 and 40 seconds in contact with oral saliva.

According to an embodiment of the invention the formulation is designed to release the content of nicotine within a period of between 30 and 60 seconds in contact with oral saliva.

In an embodiment of the invention, the formulation comprises said pH regulating agent in an amount of at least 2.7 % by weight of the formulation.

According to an embodiment of the invention, the formulation comprises said pH regulating agent in an amount of between 2.7 and 5.7 % by weight of said formulation.

In an embodiment of the invention, the formulation is designed to release the content of pH regulating agent within a period of 40 seconds in contact with oral saliva.

According to an embodiment of the invention, the formulation is designed to release the content of pH regulating agent within a period of 20 seconds in contact with oral saliva.

According to an embodiment of the invention, the formulation is designed to release the content of pH regulating agent within a period of 5 - 40 seconds in contact with oral saliva.

According to an embodiment of the invention, the formulation is designed to release the content of pH regulating agent within a period of 5 - 20 seconds in contact with oral saliva.

In an embodiment of the invention, the content of pH regulating agent is released before the content of nicotine.

In an embodiment of the invention, at least 50% by weight of the content of pH regulating agent is released before 50 % by weight of the content of nicotine.

The oral nicotine formulation is provided in an orally disintegrating tablet.

One advantage of the above embodiment may be that an orally disintegrating tablet is a highly suitable delivery vehicle for said oral nicotine formulation.

In an embodiment of the invention, the oral nicotine formulation is provided in a sublingual orally disintegrating tablet.

This is even more advantageous, given the fact that very high concentrations of nicotine may be obtained sublingually with only minimum burning in the throat. A very high sublingually uptake thus both keeps the burning at a minimum and increases the nicotine uptake at the same time.

In an embodiment of the invention, the formulation being designed to disintegrate within a period of less than 60 seconds upon oral administration.

In an embodiment of the invention, the formulation is designed for the content of nicotine to dissolve in the saliva within a period of less than 60 seconds upon oral administration.

In an embodiment of the invention, the nicotine is provided as nicotine salt.

In an embodiment of the invention, the nicotine salt is selected from nicotine ascorbate, nicotine aspartate, nicotine benzoate, nicotine monotartrate, nicotine bitartrate, nicotine chloride (e.g., nicotine hydrochloride and nicotine dihydrochloride), nicotine citrate, nicotine fumarate, nicotine gensitate, nicotine lactate, nicotine mucate, nicotine laurate, nicotine levulinate, nicotine malate nicotine perchlorate, nicotine pyruvate, nicotine salicylate, nicotine sorbate, nicotine succinate, nicotine zinc chloride, nicotine sulfate, nicotine tosylate and hydrates thereof (e.g., nicotine zinc chloride monohydrate).

In an embodiment of the invention, the nicotine salt comprises nicotine bitartrate.

In the present context, nicotine bitartrate includes hydrates thereof.

According to an embodiment of the invention, the nicotine salt is a water-soluble nicotine salt.

In the present context, the term "water-soluble salt" is understood as a salt having a solubility in water of at least 10 g of salt per 100 mL water at standard lab conditions, including temperature of 25 degrees Celsius, atmospheric pressure, and pH of 7.

Also, it should be understood that the when the nicotine comprises nicotine salt, possibly in combination with other forms of nicotine, the nicotine salt may consist of only one nicotine salt, or may be a combination of two or more nicotine salts.

According to an embodiment of the invention, the nicotine salt comprises nicotine bitartrate.

In an embodiment of the invention, the nicotine is provided as free nicotine base.

In an embodiment of the invention, nicotine is selected from the group consisting of a nicotine salt or the free base form of nicotine.

In an embodiment of the invention, said nicotine is provided as a synthetic nicotine. An advantage of the above embodiment may be that a more desirable taste profile may be obtained by avoiding undesirable taste notes that may be included in nicotine obtained from tobacco.

Disclosed but not claimed is, that said nicotine is provided as a complex between nicotine and an ion exchange resin.

Disclosed but not claimed is, that said complex between nicotine and the ion exchange resin is nicotine polacrilex resin (NPR).

Disclosed but not claimed is, that the nicotine is provided in association with a fatty acid.

Disclosed but not claimed is, that the nicotine is provided in ionic complex with at least one mucoadhesive water-soluble anionic polymer.

In an embodiment of the invention, the formulation provides a peak saliva concentration of nicotine of more than 0.3 mg/mL and a peak saliva pH of more than 8 during the first 120 seconds upon oral administration.

In an embodiment of the invention, the formulation provides a peak saliva concentration of nicotine of more than 0.4 mg/mL and a peak saliva pH of more than 8 during the first 120 seconds upon oral administration.

In an embodiment of the invention, the formulation provides a peak saliva concentration of nicotine of more than 0.5 mg/mL and a peak saliva pH of more than 8 during the first 120 seconds upon oral administration.

In embodiments where the formulation provides a peak saliva concentration of nicotine of more than 0.3 mg/mL, such as more than 0.5 mg/mL, during the first 120 seconds upon oral administration, the amount of nicotine in the formulation should be adjusted to at least the amount necessary for obtaining this. Depending on the specific formulation and delivery vehicle, the amount of nicotine in the formulation may be higher than 0.5 mg in some embodiments, such as e.g. at least 1 mg or at least 2 mg.

In an embodiment of the invention, the formulation is designed for the content of nicotine to dissolve in the oral saliva within a period of less than 60 seconds upon oral administration, and wherein at least 40% by weight of the nicotine is absorbed through the oral mucosa.

In an embodiment of the invention, the formulation is designed for the content of nicotine to dissolve in the oral saliva within a period of less than 60 seconds upon oral administration, and wherein at least 40% to less than 50% by weight of the nicotine is absorbed through the oral mucosa.

In an embodiment of the invention, the formulation is designed for the content of nicotine to dissolve in the oral saliva within a period of less than 60 seconds upon oral administration, and
- wherein at least 40% to less than 50% by weight of the nicotine is absorbed through the oral mucosa, or
- wherein at least 50% by weight of the nicotine is absorbed through the oral mucosa.

In an embodiment of the invention, the formulation is designed for the content of nicotine to dissolve in the oral saliva within a period of less than 60 seconds upon oral administration, and wherein at least 50% by weight of the nicotine is absorbed through the oral mucosa.

According to the claims, the formulation comprises at least one polyol, wherein the polyol comprises more than 40% by weight of the formulation.

In an embodiment of the invention, the formulation further comprises a disintegrant.

One advantage of the above embodiment may be that said disintegrant facilitates the disintegration and dissolution of the formulation, whereby a release of the nicotine and pH controlling agent is achieved.

In an embodiment of the invention, the formulation comprises disintegrant in an amount of 1-10 % by weight of the formulation.

According to an embodiment of the invention, the formulation comprises disintegrant in an amount of 2-8% by weight of the formulation, such as 4-6 % by weight of the formulation, such as about 5% by weight of the formulation.

Advantageously, the level of disintegrant is high enough to obtain a fast disintegration, but not too high as high amounts may increase production costs unnecessarily.

In an embodiment of the invention, the disintegrant is selected from starch, pregelatinated starch, modified starch (including potato starch, maize starch, starch 1500, sodium starch glycolate and starch derivatives), cellulose, microcrystalline cellulose, alginates, ion-exchange resin, and superdisintegrants, such as crosslinked cellulose (such as sodium carboxy methyl cellulose), crosslinked polyvinyl pyrrolidone (PVP), crosslinked starch, crosslinked alginic acid, natural superdisintegrants, and calcium silicate, and combinations thereof.

In an embodiment of the invention the disintegrant comprises cross-linked polyvinylpyrrolidone.

In an embodiment of the invention the disintegrant is cross-linked polyvinylpyrrolidone.

An advantage of using cross-linked polyvinylpyrrolidone, also known as crospovidone, as disintegrant, may be that it decreases the dependence of the disintegration time on the compression force while allowing rather low disintegration times. This may be preferred especially for fast disintegrating tablets. Also, by being more independent of compression force, a lower variation between tablets due to variations in compression force is facilitated.

In an embodiment of the invention, at least 50% by weight of the cross-linked polyvinylpyrrolidone has a particle size below 50 micrometers.

This corresponds to commercial grades of crospovidone Kollidon CL-F and CL-SF.

In an embodiment of the invention, at least 25% by weight of the cross-linked polyvinylpyrrolidone has a particle size below 15 micrometers.

This corresponds to commercial grade of crospovidone Kollidon CL-SF.

An advantage of the above embodiment of using cross-linked polyvinylpyrrolidone with a smaller particle size facilitates a shorter disintegration time, e.g. due to a larger relative surface of the disintegrant particles.

In an embodiment of the invention, the solid oral nicotine formulation is provided as a tablet having a weight of 25 to 200mg, such as 50 to 150 mg, such as 70-120mg, such as about 75 mg or about 100 mg.

An advantage of the above embodiment may be that it provides a desirable low disintegration time, while allowing a sufficiently high nicotine amount to be included in the tablet.

In an embodiment of the invention, the solid oral nicotine formulation is provided as a powdered formulation in an amount of 100 to 800 mg, such as 200 to 600 mg, such as about 400 mg.

In an embodiment of the invention, the formulation comprises sodium stearyl fumarate (SSF) as a lubricant.

An advantage of the above embodiment may be that it facilitates a shorter disintegration time of the formulation.

In an embodiment of the invention, the formulation comprises microcrystalline cellulose in an amount of 1-10 % by weight of the formulation.

An advantage of the above embodiment is that a lower friability may be obtained without compromising the mouthfeel. Including too high amounts of microcrystalline cellulose may lead to a dusty mouthfeel.

According to an embodiment of the invention, the formulation comprises microcrystalline cellulose in an amount of 2-8% by weight of the formulation, such as 4-6 % by weight of the formulation, such as about 5% by weight of the formulation.

According to an embodiment of the invention, the formulation comprises mannitol as a bulk sweetener.

According to an embodiment of the invention, the composition further comprises an amount of an insoluble composition.

An advantage of the above embodiment may be that a residue is left even after use of a pouch comprising the formulation. This may lead to a pleasant perception for users of the pouch, e.g. due to similarity with tobacco containing products.

According to an embodiment of the invention, the insoluble composition comprises at least one selected from dibasic calcium phosphate, calcium carbonate DC, mono-, diglyceride powder, hydrogenated vegetable oil, and combinations thereof.

According to an embodiment of the invention, the amount of the insoluble composition is between 5 and 50 % by weight of the formulation, such as between 10 and 30% by weight of the formulation.

Mannitol is advantageous due to a lower compactability compared to e.g. sorbitol, isomalt, and xylitol, i.e. for a given compression force a lower hardness of the tablet is obtained by using mannitol compared to sorbitol, isomalt or xylitol.

The invention further relates to a solid oral nicotine formulation for use in alleviation of nicotine craving, comprising nicotine and a pH regulating agent, wherein the formulation is designed to release the content of nicotine within a period of 60 seconds in contact with oral saliva and the formulation is designed to release the content of pH regulating agent within a period of 60 seconds in contact with oral saliva.

In the present context, it should be understood that said use in the alleviation of nicotine craving involves administering said solid oral nicotine formulation orally.

In an embodiment of the invention, the solid oral nicotine formulation according to the invention or any of its embodiments is used in alleviation of nicotine craving.

In an embodiment of the invention the nicotine is not in ionic complex with a mucoadhesive water-soluble anionic polymer.

In an embodiment of the invention the nicotine does not contain a nicotine complex.

In an embodiment of the invention, a solid oral nicotine formulation for fast onset nicotine craving relief comprises nicotine and a pH regulating agent, wherein the formulation is designed to release the content of nicotine within a period of 60 seconds in contact with oral saliva and the formulation is designed to release the content of pH regulating agent within a period of 60 seconds in contact with oral saliva and wherein the formulation comprises nicotine in an amount of at least 0.5 mg, such as nicotine in an amount of between 0.5 mg and 10.0 mg, such as between 0.5 mg and 4.0 mg.

In an embodiment of the invention, a solid oral nicotine formulation for fast onset nicotine craving relief comprises nicotine and a pH regulating agent, wherein the formulation is designed to release the content of nicotine within a period of 60 seconds in contact with oral saliva and the formulation is designed to release the content of pH regulating agent within a period of 60 seconds in contact with oral saliva and wherein the formulation comprises nicotine in an amount of at least 0.5 mg, such as nicotine in an amount of between 0.5 mg and 8 mg wherein said nicotine is provided as nicotine salt, and wherein the nicotine salt is selected from nicotine ascorbate, nicotine aspartate, nicotine benzoate, nicotine monotartrate, nicotine bitartrate, nicotine chloride (e.g., nicotine hydrochloride and nicotine dihydrochloride), nicotine citrate, nicotine fumarate, nicotine gensitate, nicotine lactate, nicotine mucate, nicotine laurate, nicotine levulinate, nicotine malate nicotine perchlorate, nicotine pyruvate, nicotine salicylate, nicotine sorbate, nicotine succinate, nicotine zinc chloride, nicotine sulfate, nicotine tosylate and hydrates thereof (e.g., nicotine zinc chloride monohydrate).

In an embodiment of the invention, a solid oral nicotine formulation for fast onset nicotine craving relief comprises nicotine and a pH regulating agent, wherein the formulation is designed to release the content of nicotine within a period of 60 seconds in contact with oral saliva and the formulation is designed to release the content of pH regulating agent within a period of 60 seconds in contact with oral saliva and wherein the formulation comprises nicotine in an amount of at least 0.5 mg, such as nicotine in wherein the formulation comprises said pH regulating agent in an amount of at least 2.7 % by weight of the formulation and wherein the nicotine is a salt in the form of nicotine bitartrate.

In an embodiment of the invention, the tablet comprises a pressed powder formulation, the tablet being designed to disintegrate within a period of less than 60 seconds upon oral administration, the powder formulation comprising an amount of nicotine salt and a pH regulating agent and wherein the tablet is designed for the content of nicotine to dissolve in the saliva within a period of less than 60 seconds upon oral administration.

In an embodiment of the invention, the tablet comprises a pressed powder formulation, the tablet being designed to disintegrate within a period of less than 60 seconds upon oral administration, the powder formulation comprising an amount of nicotine salt and a pH regulating agent and wherein the tablet is designed for the content of nicotine to dissolve within a period of less than 60 seconds upon oral administration and wherein the nicotine salt is water soluble.

In an embodiment of the invention, the orally disintegrating nicotine tablet for use in the alleviation of nicotine craving, comprising a pressed powder formulation, the tablet being designed to disintegrate within a period of less than 60 seconds upon oral administration, the powder formulation comprising an amount of nicotine and a pH regulating agent.

In the present context, it should be understood that said use in the alleviation of nicotine craving involves administering said orally disintegrating nicotine tablet orally.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration.

In an embodiment of the invention the nicotine is not in ionic complex with a mucoadhesive water-soluble anionic polymer.

In an embodiment of the invention the nicotine does not contain a nicotine complex. According to an embodiment of the invention, the orally disintegrating nicotine tablet for nicotine craving relief comprises a pressed powder formulation, the tablet being designed to disintegrate within a period of less than 60 seconds upon oral administration, the powder formulation comprising an amount of nicotine and a pH regulating agent, and wherein the tablet is designed for the content of nicotine to dissolve in the saliva within a period of less than 60 seconds upon oral administration.

According to an embodiment of the invention, the orally disintegrating nicotine tablet for nicotine craving relief comprises a pressed powder formulation, the tablet being designed to disintegrate within a period of less than 60 seconds upon oral administration, the powder formulation comprising an amount of nicotine and a pH regulating agent, wherein the tablet is designed for the content of nicotine to dissolve in the saliva within a period of less than 60 seconds upon oral administration and wherein the nicotine is provided as a nicotine salt. This salt should preferably be a water-soluble salt.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration and wherein the tablet comprises nicotine in an amount of between 0.5 mg to 4.0 mg.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein the tablet comprises nicotine in an amount of between 0.5 mg to 4 mg administration and wherein the nicotine is provided as a nicotine salt. This salt should preferably be a water-soluble salt.

According to an embodiment of the invention, the orally disintegrating nicotine sublingual tablet for nicotine craving relief comprises a pressed powder formulation, the tablet being designed to disintegrate within a period of less than 60 seconds upon oral administration, the powder formulation comprising an amount of nicotine and a pH regulating agent, and wherein the tablet is designed for the content of nicotine to dissolve in the saliva within a period of less than 60 seconds upon oral administration.

According to an embodiment of the invention, the orally disintegrating nicotine sublingual tablet for nicotine craving relief comprises a pressed powder formulation, the tablet being designed to disintegrate within a period of less than 60 seconds upon oral administration, the powder formulation comprising an amount of nicotine and a pH regulating agent, wherein the tablet is designed for the content of nicotine to dissolve in the saliva within a period of less than 60 seconds upon oral administration and wherein the nicotine is provided as a nicotine salt. This salt should preferably be a water-soluble salt.

In an embodiment of the invention, the orally disintegrating sublingual nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration and wherein the tablet comprises nicotine in an amount of between 0.5 mg to 4.0 mg.

In an embodiment of the invention, the orally disintegrating sublingual nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein the tablet comprises nicotine in an amount of between 0.5 mg to 4.0 mg administration and wherein the nicotine is provided as a nicotine salt. This salt should preferably be a water-soluble salt.

According to an embodiment of the invention, the orally disintegrating nicotine tablet for nicotine craving relief comprises a pressed powder formulation, the tablet being designed to disintegrate within a period of less than 60 seconds upon oral administration, the powder formulation comprising an amount of nicotine and a pH regulating agent, and wherein the tablet is designed for the content of nicotine to dissolve in the saliva within a period of less than 60 seconds upon oral administration and wherein the pressed powder comprises at least one polyol and wherein the polyol comprises more than 50% by weight of the tablet.

According to an embodiment of the invention, the orally disintegrating nicotine tablet for nicotine craving relief comprises a pressed powder formulation, the tablet being designed to disintegrate within a period of less than 60 seconds upon oral administration, the powder formulation comprising an amount of nicotine and a pH regulating agent, wherein the tablet is designed for the content of nicotine to dissolve in the saliva within a period of less than 60 seconds upon oral administration, wherein the pressed powder comprises at least one polyol and wherein the polyol comprises more than 50% by weight of the tablet and wherein the nicotine is provided as a nicotine salt. This salt should preferably be a water-soluble salt.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein the pressed powder comprises at least one polyol and wherein the polyol comprises more than 50% by weight of the tablet and wherein the tablet comprises nicotine in an amount of between 0.5 mg to 4.0 mg.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein the pressed powder comprises at least one polyol and wherein the polyol comprises more than 50% by weight of the tablet and wherein the tablet comprises nicotine in an amount of between 0.5 mg to 4.0 mg administration and wherein the nicotine is provided as a nicotine salt. This salt should preferably be a water-soluble salt.

In an embodiment of the invention, the tablet comprises a pressed powder formulation, the tablet being designed to disintegrate within a period of less than 60 seconds upon oral administration, the powder formulation comprising an amount of nicotine salt and a pH regulating agent and wherein the tablet is designed for the content of nicotine to dissolve in the saliva within a period of less than 60 seconds upon oral administration.

In an embodiment of the invention, the tablet comprises a pressed powder formulation, the tablet being designed to disintegrate within a period of less than 60 seconds upon oral administration, the powder formulation comprising an amount of nicotine salt and a pH regulating agent and wherein the tablet is designed for the content of nicotine to dissolve within a period of less than 60 seconds upon oral administration and wherein the nicotine salt is water soluble.

In an embodiment of the invention, the orally disintegrating nicotine tablet for use in the alleviation of nicotine craving, comprising a pressed powder formulation, the tablet being designed to disintegrate within a period of less than 60 seconds upon oral administration, the powder formulation comprising an amount of nicotine and a pH regulating agent.

In the present context, it should be understood that said use in the alleviation of nicotine craving involves administering said orally disintegrating nicotine tablet orally.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration.

In an embodiment of the invention the nicotine is not in ionic complex with a mucoadhesive water-soluble anionic polymer.

In an embodiment of the invention the nicotine does not contain a nicotine complex.

According to an embodiment of the invention, the orally disintegrating nicotine tablet for nicotine craving relief comprises a pressed powder formulation, the tablet being designed to disintegrate within a period of less than 60 seconds upon oral administration, the powder formulation comprising an amount of nicotine and a pH regulating agent, and wherein the tablet is designed for the content of nicotine to dissolve in the saliva within a period of less than 60 seconds upon oral administration and wherein the formulation is designed to release the content of nicotine within a period of 60 seconds in contact with oral saliva.

According to an embodiment of the invention, the orally disintegrating nicotine tablet for nicotine craving relief comprises a pressed powder formulation, the tablet being designed to disintegrate within a period of less than 60 seconds upon oral administration, wherein the formulation is designed to release the content of nicotine within a period of 60 seconds in contact with oral saliva the powder formulation comprising an amount of nicotine and a pH regulating agent, wherein the tablet is designed for the content of nicotine to dissolve in the saliva within a period of less than 60 seconds upon oral administration and wherein the nicotine is provided as a nicotine salt. This salt should preferably be a water-soluble salt.

In an embodiment of the invention, the orally disintegrating nicotine tablet comprises a pressed powder formulation, the tablet being designed to disintegrate within a period of less than 60 seconds upon oral administration, wherein the formulation is designed to release the content of nicotine within a period of 60 seconds in contact with oral saliva, the powder formulation comprising an amount of nicotine salt and a pH regulating agent, wherein the tablet wherein the tablet is designed for the content of nicotine to dissolve within a period of less than 60 seconds upon oral administration and wherein the tablet comprises nicotine in an amount of between 0.5 mg to 4.0 mg. In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein the formulation is designed to release the content of nicotine within a period of 60 seconds in contact with oral saliva and wherein the tablet comprises nicotine in an amount of between 0.5 mg to 4.0 mg administration and wherein the nicotine is provided as a nicotine salt. This salt should preferably be a water-soluble salt.

According to an embodiment of the invention, the orally disintegrating nicotine sublingual tablet for nicotine craving relief comprises a pressed powder formulation, the tablet being designed to disintegrate within a period of less than 60 seconds upon oral administration, the powder formulation comprising an amount of nicotine and a pH regulating agent, and wherein the tablet is designed for the content of nicotine to dissolve in the saliva within a period of less than 60 seconds upon oral administration and wherein the formulation is designed to release the content of nicotine within a period of 60 seconds in contact with oral saliva.

According to an embodiment of the invention, the orally disintegrating nicotine sublingual tablet for nicotine craving relief comprises a pressed powder formulation, the tablet being designed to disintegrate within a period of less than 60 seconds upon oral administration, the powder formulation comprising an amount of nicotine and a pH regulating agent, wherein the formulation is designed to release the content of nicotine within a period of 60 seconds in contact with oral saliva , wherein the tablet is designed for the content of nicotine to dissolve in the saliva within a period of less than 60 seconds upon oral administration and wherein the nicotine is provided as a nicotine salt. This salt should preferably be a water-soluble salt.

In an embodiment of the invention, the orally disintegrating sublingual nicotine tablet comprises a pressed powder formulation, the tablet being designed to disintegrate within a period of less than 60 seconds upon oral administration, wherein the formulation is designed to release the content of nicotine within a period of 60 seconds in contact with oral saliva, the powder formulation comprising an amount of nicotine salt and a pH regulating agent, wherein the tablet wherein the tablet is designed for the content of nicotine to dissolve within a period of less than 60 seconds upon oral administration and wherein the tablet comprises nicotine in an amount of between 0.5 mg to 4.0 mg.

In an embodiment of the invention, the orally disintegrating sublingual nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein the formulation is designed to release the content of nicotine within a period of 60 seconds in contact with oral saliva, wherein the tablet comprises nicotine in an amount of between 0.5 mg to 4.0 mg administration and wherein the nicotine is provided as a nicotine salt. This salt should preferably be a water-soluble salt.

According to an embodiment of the invention, the orally disintegrating nicotine tablet for nicotine craving relief comprises a pressed powder formulation, the tablet being designed to disintegrate within a period of less than 60 seconds upon oral administration, the powder formulation comprising an amount of nicotine and a pH regulating agent, and wherein the tablet is designed for the content of nicotine to dissolve in the saliva within a period of less than 60 seconds upon oral administration, wherein the formulation is designed to release the content of nicotine within a period of 60 seconds in contact with oral saliva and wherein the pressed powder comprises at least one polyol and wherein the polyol comprises more than 50% by weight of the tablet.

According to an embodiment of the invention, the orally disintegrating nicotine tablet for nicotine craving relief comprises a pressed powder formulation, the tablet being designed to disintegrate within a period of less than 60 seconds upon oral administration, wherein the formulation is designed to release the content of nicotine within a period of 60 seconds in contact with oral saliva, the powder formulation comprising an amount of nicotine and a pH regulating agent, wherein the tablet is designed for the content of nicotine to dissolve in the saliva within a period of less than 60 seconds upon oral administration, wherein the pressed powder comprises at least one polyol and wherein the polyol comprises more than 50% by weight of the tablet and wherein the nicotine is provided as a nicotine salt. This salt should preferably be a water-soluble salt.

In an embodiment of the invention, the orally disintegrating nicotine tablet comprises a pressed powder formulation, the tablet being designed to disintegrate within a period of less than 60 seconds upon oral administration, wherein the formulation is designed to release the content of nicotine within a period of 60 seconds in contact with oral saliva, the powder formulation comprising an amount of nicotine salt and a pH regulating agent, wherein the tablet wherein the tablet is designed for the content of nicotine to dissolve within a period of less than 60 seconds upon oral administration, wherein the pressed powder comprises at least one polyol and wherein the polyol comprises more than 50% by weight of the tablet and wherein the tablet comprises nicotine in an amount of between 0.5 mg to 4.0 mg.

In an embodiment of the invention, the orally disintegrating nicotine tablet comprises a pressed powder formulation, the tablet being designed to disintegrate within a period of less than 60 seconds upon oral administration, the powder formulation comprising an amount of nicotine salt and a pH regulating agent, wherein the tablet wherein the tablet is designed for the content of nicotine to dissolve within a period of less than 60 seconds upon oral administration, wherein formulation is designed to release the content of nicotine within a period of 60 seconds in contact with oral saliva, wherein the pressed powder comprises at least one polyol and wherein the polyol comprises more than 50% by weight of the tablet and wherein the tablet comprises nicotine in an amount of between 0.5 mg to 4.0 mg administration and wherein the nicotine is provided as a nicotine salt. This salt should preferably be a water-soluble salt.

Moreover, the invention relates to a composition for use in a method of alleviation of nicotine craving by administering an effective amount of said solid oral nicotine formulation according to the invention or any of the embodiments.

### DETAILED DESCRIPTION

As used herein, the term "orally disintegrating tablet" refers to a tablet for oral administering which disintegrates in the oral cavity relatively fast from the administering, such as within about three minutes from oral administering. Orally disintegrating tablets may be intended for use as a sublingual tablet for positioning under the tongue, as a buccal tablet, as a tablet for melting on the tongue, or for other types of oral administering.

Orally disintegrating tablets may also be referred to "orally dissolving tablets", and these two terms are used interchangeably herein. Commonly, these terms are also referred to by their abbreviation, ODT. Similarly, the terms "fast dissolving tablet" and "fast disintegrating tablet", as well as the abbreviation FDT, refers herein to an orally disintegrating tablet.

As used herein, the term "disintegrate" refers to a reduction of a said object to components, fragments or particles. Disintegration time is measured in vitro. The in vitro measurements are carried out in accordance to European Pharmacopeia 9.0, section 2.9.1, Disintegration of tablets and capsules.

As used herein, the term "dissolve" is the process where a solid substance enters a solvent (oral saliva) to yield a solution. Unless otherwise stated, dissolving implies a full dissolving of the compound in question.

As used herein, the terms "disintegrant" refers to an ingredient facilitating disintegration of an orally disintegrating tablet, when the orally disintegrating tablet comes into contact with saliva. Disintegrants usable within the scope of the invention may include starch, pregelatinated starch, modified starch (including potato starch, maize starch, starch 1500, sodium starch glycolate and starch derivatives), cellulose, microcrystalline cellulose, alginates, ion-exchange resin, and superdisintegrants, such as crosslinked cellulose (such as sodium carboxy methyl cellulose), crosslinked polyvinyl pyrrolidone (PVP), crosslinked starch, crosslinked alginic acid, natural superdisintegrants, and calcium silicate. Disintegrants may often be considered as measure promoting the break-up of the dosage form into smaller fragments upon administration to allow the onset of drug dissolution and eventual absorption.

As used herein, the term "nicotine" refers to nicotine in any form, including free base nicotine, nicotine salts, but not nicotine bound to ion exchange resins, such as nicotine polacrilex, nicotine bound to zeolites; nicotine bound to cellulose, such as microcrystalline cellulose, such as of microbial origin, or starch microspheres, nicotine bound to CaCO3, and mixtures thereof. Thus, when referring to nicotine amounts, the amounts refers to the amount of pure nicotine. Thus, when measuring the concentration of nicotine added as nicotine salt, it is the mass of the equivalent amount of pure nicotine, not the mass of the salt, that is relevant. Nicotine also covers nicotine not obtained from tobacco, often referred to as synthetic nicotine.

As used herein, the term "nicotine salt" refers to nicotine in ionized form bonded electrostatically to a counterion.

As used herein, the term "NBT" refers to nicotine bitartrate and hydrates thereof.

As used herein, the term "%" and "percent" refers to percent by weight, unless otherwise is stated.

As used herein, the term "release of nicotine" refers to the nicotine being made bioavailable, i.e. available for absorption over the mucous membrane in the oral cavity. While some forms of nicotine require dissolution for being bioavailable, other forms may be readily absorbed into the body without dissolution. For example, in order for the nicotine to be bioavailable, the matrix of the solid formulation should be disintegrated. Some forms of nicotine require the nicotine to further be released from e.g. a carrier, e.g. nicotine from a nicotine-ion exchange resin such as nicotine polacrilex. Other nicotine forms, such nicotine salts, hereunder nicotine bitartrate, may readily dissolve upon disintegration of the matrix of the solid formulation. Still, some nicotine forms may not require dissolving. This applies for e.g. nicotine free base, which is released upon disintegration of the solid formulation matrix.

As used herein, the term "peak saliva concentration of nicotine" refers to the peak value of the concentration of nicotine in saliva of the oral cavity, where the saliva includes delivery vehicle of the nicotine dissolved therein. Also, it should be understood that the peak saliva concentration is considered to be achieved whenever the criterion is fulfilled. E.g. if a peak saliva concentration of nicotine is at least 0.5 mg/mL, this peak saliva concentration is achieved whenever the concentration of nicotine exceeds 0.5 mg/mL. Measurements of peak saliva nicotine concentration is performed as follows:
One dosage of the formulation is administered sublingually to at least six individuals. At specified time intervals, the saliva is collected. The experiment is repeated. Thus, each nicotine concentration value is the arithmetic mean of 12 measurements, i.e. performed on saliva-samples from six individuals times 2. The nicotine concentration of saliva is analyzed on HPLC after extraction into relevant buffer.

As used herein, the term "peak saliva pH" refers to the peak value of the pH in saliva of the oral cavity, where the saliva includes any delivery vehicle of the pH regulating agent. Also, it should be understood that the peak saliva pH is considered to be achieved whenever the criterion is fulfilled. E.g. if a peak saliva pH is at least 7.5, this peak saliva pH is achieved whenever the pH exceeds 7.5. Peak saliva pH is measured in vivo and is measured as follows:
At least 6 individuals chewed on a gum base free of buffer for 1 minute, after which the initial pH in a sample from the saliva from each of the individuals is measured with a suitable pH-electrode system, e.g. a stainless steel electrode PHW77-SS. Only individuals having, after chewing on a gum base free of buffer for one minute, an initial pH in the saliva inside the range from 6.7 and 7.3 are selected. These individuals thereby qualify as average individuals.

One dosage of the formulation is administered sublingually to at least six individuals. Hereafter, the saliva pH from each of the six individuals is measured at specified time intervals. Thus, each pH-value is the arithmetic mean of six measurements performed on saliva-samples from six individuals.

As used herein, the term "pH regulating agent" refers to agents, which active adjust and regulates the pH value of the solution to which they have been added or are to be added. Thus, pH regulating agents may be acids and bases, including acidic buffering agents and alkaline buffering agents. On the other hand, pH regulating agents does not including substances and compositions that can only affect the pH by dilution. Furthermore, pH regulating agents does not include e.g. flavoring, fillers, etc.

As used herein, the term "buffering agent" is used interchangeably with "buffer" and refers to agents for obtaining a buffer solution. Buffering agents include acidic buffering agents, i.e. for obtaining a buffer solution with an acidic pH, and alkaline buffering agents, i.e. for obtaining a buffer solution with an alkaline pH.

As used herein, the term "fast onset nicotine craving relief" refers to relief of nicotine craving, for which the onset is relatively fast, i.e. only a relatively short period of time after oral administering. In embodiments of the invention, the fast onset refers to a period after oral administration until craving relief is experienced being no more than 180 seconds, such as no more than 120 seconds, such as no more than 60 seconds.

### EXAMPLES

The following non-limiting examples illustrate different variations of the present invention. Only samples which meet all the requirements of the claims are within the scope of the invention. Any other samples are to be understood as for illustrative purposes only.

### Example 1

### Preparation of fast disintegrating tablet

In the present example six fast disintegrating tablets (FDT) with 1 mg nicotine are prepared with formulations as outlined in table 1. The fast disintegrating tablet is prepared with NBT (nicotine bitartrate). Punch used: 7.00 mm, circular, shallow concave, D tooling. Tablet weight: 100.0 mg.

**Table 1 - Fast disintegrating tablet compositions. Amounts are given in mg. FDT=Fast disintegrating tablet.**

| | **FDT(a)** | **FDT(b)** | **FDT(c)** | **FDT(d)** | **FDT(e)** | **FDT(f)** |
|---|---|---|---|---|---|---|
| NBT | 2.849 | 2.849 | 2.849 | 2.849 | 2.849 | 2.849 |
| Microcrystalline cellulose | - | - | - | 40.175 | 40.175 | 40.175 |
| Mannitol | 81.351 | 81.351 | 81.351 | 40.175 | 40.175 | 40.175 |
| Crospovidone | 5.0 | - | - | 5.0 | - | - |
| Croscarmellose Sodium | - | 5.0 | - | - | 5.0 | - |
| Sodium Starch Glycolate | - | - | 5.0 | - | - | 5.0 |
| Peppermint | 4.0 | 4.0 | 4.0 | 4.0 | 4.0 | 4.0 |
| Menthol | 0.4 | 0.4 | 0.4 | 0.4 | 0.4 | 0.4 |
| Sucralose | 0.4 | 0.4 | 0.4 | 0.4 | 0.4 | 0.4 |
| Sodium carbonate | 5.0 | 5.0 | 5.0 | 5.0 | 5.0 | 5.0 |
| Silicium dioxide | - | - | - | 1.0 | 1.0 | 1.0 |
| Magnesium stearate | 1.0 | 1.0 | 1.0 | 1.0 | 1.0 | 1.0 |
| Total | 100.0 | 100.0 | 100.0 | 100.0 | 100.0 | 100.0 |

Raw materials are weighed from bags or buckets into separate weighing containers.

All excipients are sifted through an 800 micrometer sieve into a stainless steel or plastic bin in the following order:
- Half the filler / bulk sweetener
- The API and all other excipients, except magnesium stearate
- The remaining half of the filler / bulk sweetener

These are mixed in a Turbula mixer for 4 - 10 minutes at 25 RPM. Then lubricant, for example magnesium stearate is sifted through an 800 micrometer sieve into the mixing bin, and the lubrication is conducted by additional mixing for 1 - 2 minutes at 25 RPM. The fill level of the mixing bin is kept between 40% and 70%, according to standardized practice. The lubricated powder blend is transferred to the hopper of a tableting machine.

The fast disintegrating tablets are manufactured on a lab scale machine, for example RIVA Piccola bi-layer tablet press. The tablet machine is commissioned by adjusting the fill depth and compression force so the weight and hardness of lozenges match the acceptance criteria. A pre-compression force could be included to avoid capping.

**Table 2: Suggested start up parameters. *The design of punches is not fixed. As the curvature impacts thickness, the thickness is not a fixed target at this time of development.**

| **Parameter** | **Target value** |
|---|---|
| Speed | 10 - 20 rpm |
| Weight of FDT | 100 mg +/- 5 % |
| Compression force | 2-8kN |
| Thickness | N/A* |
| Friability (100rpm) | < 1 % |

The acceptance criteria for friability should be fulfilled so packaging of the resulting fast disintegrating tablets is possible, but in this embodiment, the bulk sweetener and or filler should have relatively good compressibility and still have fast disintegration. The fast disintegrating tablets according to the invention may comprise coloring agents. According to an embodiment of the invention, the fast disintegrating tablets may comprise color agents and whiteners such as FD&C-type dyes and lakes, fruit and vegetable extracts, titanium dioxide and combinations thereof.

### Example 2

### Preparation of fast disintegrating tablet using ready to use systems

Another way of preparing fast disintegrating tablets would be to use a ready to use system. Suitable for the purpose could be but not limited to: Pearlitol Flash (Roquette), Pharmaburst 500 (SPI Pharma), Ludiflash (BASF), ProSolv (JRS Pharma), ProSolv EasyTab (JRS Pharma), F-Melt (Fuji Chemical), SmartEx50 or SmartEx100 (Shin Etsu / Harke Pharma). These ready to use systems co-processed systems where filler, disintegrant, glidant or similar are implemented in the one powder mix. This saves handling of several excipients and ensures homogeneity between excipients.

In the present example five fast disintegrating tablets (FDT(g) - FDT(k)) without nicotine are prepared with ready to use systems in formulations as outlined in table 3A. The fast disintegrating tablet is prepared without NBT (placebo). Adding nicotine to the fast disintegrating tablets is expected to influence disintegration time only insignificantly.

In this example, the following conditions where applied. Punch used: 7.00 mm, circular, shallow concave, B tooling. Tablet weight: 100.0 mg.

**Table 3A - Fast disintegrating tablet compositions with different ready to use systems. Amounts are given in mg. FDT=Fast disintegrating tablet.**

| | **FDT(g)** | **FDT(h)** | **FDT(i)** | **FDT(j)** | **FDT(k)** |
|---|---|---|---|---|---|
| Ludiflash | 81.7 | - | - | - | - |
| Pearlitol Flash | - | 81.7 | - | - | - |
| SmartEx QD50 | - | - | 81.7 | - | - |
| F-Melt | - | - | - | 83.7 | - |
| ProSolv ODT G2 | - | - | - | - | 83.7 |
| Peppermint | 4.4 | 4.4 | 4.4 | 4.4 | 4.4 |
| Menthol | 1.5 | 1.5 | 1.5 | 1.5 | 1.5 |
| Sucralose | 0.4 | 0.4 | 0.4 | 0.4 | 0.4 |
| Sodium carbonate | 5.0 | 5.0 | 5.0 | 5.0 | 5.0 |
| Crospovidone | 5.0 | 5.0 | 5.0 | - | - |
| Croscarmellose Sodium | - | - | - | 3.0 | - |
| Sodium Starch Glycolate | - | - | - | - | 3.0 |
| Magnesium stearate | 2.0 | 2.0 | 2.0 | 2.0 | 2.0 |
| Total | 100.0 | 100.0 | 100.0 | 100.0 | 100.0 |

Additionally, five fast disintegrating tables (FDT(l) - FDT(p)) with nicotine are prepared with ready to use systems in formulations as outlined in table 3B.

In this example, the following conditions where applied. Punch used: 7.00 mm, circular, shallow concave, B tooling. Tablet weight: 100.0 mg.

**Table 3B - Fast disintegrating tablet compositions with different ready to use systems and nicotine as nicotine bitartrate, NBT, or nicotine polacrilex, NPR (15% nicotine load). Amounts are given in mg. FDT=Fast disintegrating tablet.**

| | **FDT(l)** | **FDT(m)** | **FDT(n)** | **FDT(o)** | **FDT(p)** |
|---|---|---|---|---|---|
| NBT | - | - | 3.0 | 3.0 | 3.0 |
| NPR | 6.7 | 6.7 | - | - | - |
| Ludiflash | 75.0 | - | - | - | - |
| Pearlitol Flash | - | 75.0 | - | - | - |
| SmartEx QD50 | - | - | 78.7 | - | - |
| F-Melt | - | - | - | 80.7 | - |
| ProSolv ODT G2 | - | - | - | - | 80.7 |
| Peppermint | 4.4 | 4.4 | 4.4 | 4.4 | 4.4 |
| Menthol | 1.5 | 1.5 | 1.5 | 1.5 | 1.5 |
| Sucralose | 0.4 | 0.4 | 0.4 | 0.4 | 0.4 |
| Sodium carbonate | 5.0 | 5.0 | 5.0 | 5.0 | 5.0 |
| Crospovidone | 5.0 | 5.0 | 5.0 | - | - |
| Croscarmellose Sodium | - | - | - | 3.0 | - |
| Sodium Starch Glycolate | - | - | - | - | 3.0 |
| Magnesium stearate | 2.0 | 2.0 | 2.0 | 2.0 | 2.0 |
| Total | 100.0 | 100.0 | 100.0 | 100.0 | 100.0 |

Further four fast disintegrating tablets (FDT(1) - FDT(4)) with nicotine are prepared with varying amounts of MCC (microcrystalline cellulose) as filler, as outlined in table 3C.

In this example, the following conditions where applied. Punch used: 7.00 mm, circular, shallow concave, B tooling. Tablet weight: 100.0 mg.

**Table 3C - Fast disintegrating tablet compositions with varying amounts of MCC and nicotine (1 mg/tablet) sorbed onto calcium carbonate, (synthetic free nicotine base sorbed onto calcium carbonate in a weight ratio of 1:2). Amounts are given in mg. FDT=Fast disintegrating tablet.**

| | **FDT(1)** | **FDT(2)** | **FDT(3)** | **FDT(4)** |
|---|---|---|---|---|
| Nicotine-calcium carbonate | 3.0 | 3.0 | 3.0 | 3.0 |
| Microcrystalline cellulose, MCC | 0.0 | 5.0 | 10.0 | 20.0 |
| Mannitol | 79.7 | 74.7 | 69.7 | 59.7 |
| Crospovidone | 5.0 | 5.0 | 5.0 | 5.0 |
| Peppermint | 4.4 | 4.4 | 4.4 | 4.4 |
| Menthol | 1.5 | 1.5 | 1.5 | 1.5 |
| Sucralose | 0.4 | 0.4 | 0.4 | 0.4 |
| Sodium carbonate | 5.0 | 5.0 | 5.0 | 5.0 |
| Magnesium stearate | 1.0 | 1.0 | 1.0 | 1.0 |
| Total | 100.0 | 100.0 | 100.0 | 100.0 |

Four fast disintegrating tablets, FDT(5) - FDT(8), with nicotine are prepared with varying amounts of disintegrant, as outlined in table 3D.

In this example, the following conditions where applied. Punch used: 7.00 mm, circular, shallow concave, B tooling. Tablet weight: 100.0 mg.

**Table 3D - Fast disintegrating tablet compositions with varying amount of disintegrant. Amounts are given in mg. FDT=Fast disintegrating tablet.**

| | **FDT(5)** | **FDT(6)** | **FDT(7)** | **FDT(8)** |
|---|---|---|---|---|
| NBT | 3.0 | 3.0 | 3.0 | 3.0 |
| Mannitol | 41.7 | 39.2 | 41.7 | 31.7 |
| Microcrystalline cellulose | 43 | 43 | 43 | 43 |
| Crospovidone | 0.0 | 2.5 | 5.0- | 10.0 |
| Peppermint | 4.4 | 4.4 | 4.4 | 4.4 |
| Menthol | 1.5 | 1.5 | 1.5 | 1.5 |
| Sucralose | 0.4 | 0.4 | 0.4 | 0.4 |
| Sodium carbonate | 5.0 | 5.0 | 5.0 | 5.0 |
| Magnesium stearate | 1.0 | 1.0 | 1.0 | 1.0 |
| Total | 100.0 | 100.0 | 100.0 | 100.0 |

Three fast disintegrating tablets, FDT(9) - FDT(11), with nicotine are prepared with varying types of lubricants, as outlined in table 3E.

In this example, the following conditions where applied. Punch used: 7.00 mm, circular, shallow concave, B tooling. Tablet weight: 100.0 mg.

**Table 3E - Fast disintegrating tablet compositions. Amounts are given in mg. FDT=Fast disintegrating tablet.**

| | **FDT(9)** | **FDT(10)** | **FDT(11)** |
|---|---|---|---|
| NBT | 3.0 | 3.0 | 3.0 |
| Microcrystalline cellulose | 5 | 5 | 5 |
| Mannitol | 78.6 | 77.6 | 77.6 |
| Crospovidone | 5.0 | 5.0 | 5.0 |
| Eucamenthol Flavour | 2 | 2 | 2 |
| Sucralose | 0.4 | 0.4 | 0.4 |
| Sodium carbonate | 5.0 | 5.0 | 5.0 |
| Magnesium stearate | 1.0 | - | - |
| Sodium stearyl fumarate | - | 2.0 | - |
| Compritol HD5 | - | - | 2.0 |
| Total | 100.0 | 100.0 | 100.0 |

Three fast disintegrating tablets, FDT(12) - FDT(14), with nicotine are prepared, as outlined in table 3F.

In this example, the following conditions where applied. Punch used: 7.00 mm, circular, shallow concave, B tooling. Tablet weight: 75.0 mg.

**Table 3F - Fast disintegrating tablet compositions. Amounts are given in mg. FDT=Fast disintegrating tablet. FDT(13) was made similar to FDT(12) but without buffer. FDT(14) was made similar to FDT(12) but without disintegrant.**

| | **FDT(12)** | **FDT(13)** | **FDT(14)** |
|---|---|---|---|
| SmartEx QD 50 | 60.0 | 65.0 | 65.0 |
| Nicotine Bitartrate (NBT) | 3.0 | 3.0 | 3.0 |
| Sodium carbonate anhydrous | 5.0 | - | 5.0 |
| Crospovidone (Kollidon CL-F, BASF) | 5.0 | 5.0 | - |
| Peppermint Powder | 0.4 | 0.4 | 0.4 |
| Sucralose | 0.4 | 0.4 | 0.4 |
| Aerosil 200 (silicium dioxide) | 0.2 | 0.2 | 0.2 |
| Magnesium Stearate | 1.0 | 1.0 | 1.0 |
| Total | 75.0 | 75.0 | 75.0 |

FDT(12)-FDT(13) were pressed to a hardness of 15-20 N. FDT (14) was pressed to a hardness of 25-35 N.

An oral pouch is prepared comprising a powdered composition, PPC 1, as outlined in table 3G. The pouch is made as follows.

The nicotine-resin complex used herein is made by mixing water, nicotine, resin (Amberlite^{®}IRP64) and glycerin. When a homogeneous solution is obtained and all nicotine has been bound by the ion exchange resin the pressure is reduced and the obtained mixture is concentrated in vacuum at elevated temperature affording the desired complex as a powder. The nicotine-resin complex is sieved.

Any cationic ion exchange resin complex (preferable a non-ionic pharmaceutical grade resin) may in principle be used. The resin is capable of binding anionic molecules at the ion exchange sites.

The obtained nicotine-resin complex powder is mixed using a Turbula mixer for 6 minutes (speed 49 rpm) with the remaining ingredients to obtain a final powder composition.

The final powder composition is filled into pouches (target fill weight 400 mg powder per pouch). The following pouch, made from long fiber paper, is used.

The material of the pouches is heat sealable non-wowen cellulose, such as long fiber paper. Pouches that are not in form of non-woven cellulose fabric may also be used according to the invention.

The powder is filled into pouches and is maintained in the pouch by a sealing.

When including smaller amounts of further humectants, apart from e.g. sugar alcohols, these further humectants are added in the same manner as magnesium stearate.

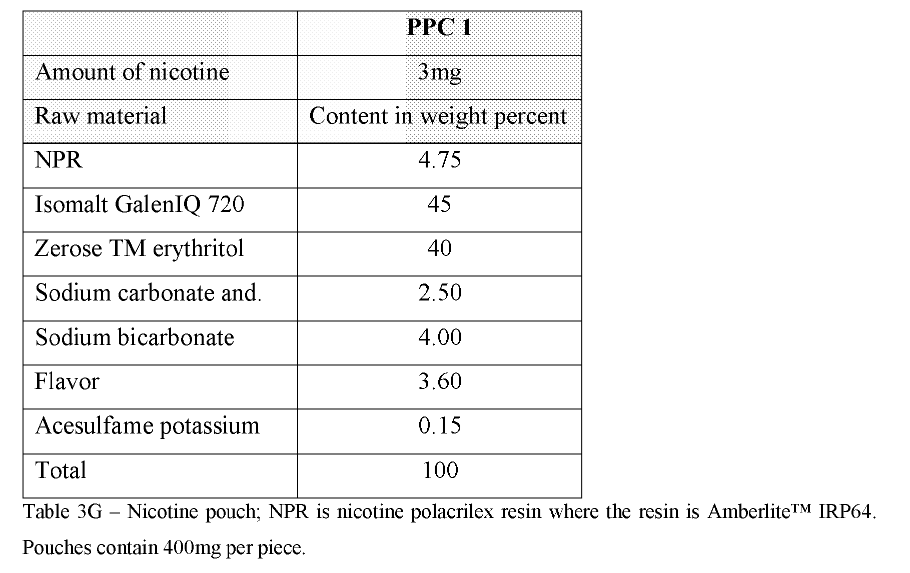

Preparation of fast dissolving pouches with residue containing nicotine polacrilex resin (NPR) or nicotine bitartrate (NBT) are prepared comprising powdered compositions, PPC 2-7, as outlined in table 3H.

Preparation of fast dissolving pouches without residue containing nicotine polacrilex resin (NPR) or nicotine bitartrate (NBT) are prepared comprising powdered compositions, PPC 8-13, as outlined in table 3I.

The pouches are made as follows.

For PPC 6, and 8-10 a method corresponding to that used for PPC 1 was used.

For PPC 2-5, 7 and 11-13, the below method was used.

Nicotine bitartrate xH2O is mixed using a Turbula mixer for 6 minutes (speed 49 rpm) with the remaining ingredients to obtain a final powder composition.

The final powder composition is filled into pouches (target fill weight 400 mg powder per pouch). The pouch material of example 2, made from long fiber paper, is used. The powder is filled into pouches and is maintained in the pouch by a sealing.

The material of the pouches is heat sealable non-wowen cellulose, such as long fiber paper. Pouches that are not in form of non-woven cellulose fabric may also be used according to the invention.

The powder is filled into pouches and is maintained in the pouch by a sealing.

When including smaller amounts of further humectants, apart from e.g. sugar alcohols, these further humectants are added in the same manner as magnesium stearate.

**Table 3H. Nicotine pouch; NPR is nicotine polacrilex resin where the resin is Amberlite^{™} IRP64. NBT is nicotine bitartrate. Pouches contain 400mg per piece.**

| **PPC** | **2** | **3** | **4** | **5** | **6** | **7** |
|---|---|---|---|---|---|---|
| Amount of nicotine | 3mg | 3mg | 3mg | 3mg | 3mg | 3mg |
| Raw material | Content in weight percent | | | | | |
| NPR | - | - | - | - | 4.75 | - |
| NBT | 2.14 | 2.14 | 2.14 | 2.14 | - | 2.14 |
| Isomalt GalenIQ 720 | 67.7 | - | 67.7 | - | 62.6 | 67.7 |
| Maltitol SweetPearl 300 DC | - | 67.7 | - | 65.2 | - | - |
| Sodium carbonate | 2.50 | 2.50 | 2.50 | 2.50 | 2.50 | 2.50 |
| Sodium bicarbonate | 4.00 | 4.00 | 4.00 | 4.00 | 4.00 | 4.00 |
| Peppermint flavor | 2.50 | 2.50 | 2.50 | 2.50 | 2.50 | - |
| Menthol | 1.00 | 1.00 | 1.00 | - | - | - |
| Eucalyptos flavor | - | - | - | 3.50 | 3.50 | 3.50 |
| Acesulfame potassium | 0.16 | 0.16 | 0.16 | 0.16 | 0.15 | 0.16 |
| Dibasic Calcium Phosphate anhydr. | 20.00 | 20.00 | - | - | - | - |
| Calcium carbonate DC | - | - | 20.00 | 20.00 | - | - |
| Mono-, diglyceride powder (emulsifier) | - | - | - | - | 10.00 | 10.00 |
| Hydrogenated Vegetable oil (solid state) | - | - | - | - | 10.00 | 10.00 |
| Total | 100 | 100 | 100 | 100 | 100 | 100 |

**Table 31. Nicotine pouch; NPR is nicotine polacrilex resin where the resin is Amberlite^{™} IRP64. NBT is nicotine bitartrate. Pouches contain 400mg per piece.**

| **PPC** | **8** | **9** | **10** | **11** | **12** | **13** |
|---|---|---|---|---|---|---|
| Amount of nicotine | 3mg | 3mg | 3mg | 3mg | 3mg | 3mg |
| Raw material | Content in weight percent | | | | | |
| NPR | 4.75 | 4.75 | 4.75 | - | - | - |
| NBT | - | - | - | 2.14 | 2.14 | 2.14 |
| Isomalt GalenIQ 720 | - | 85.1 | - | - | 87.7 | - |
| Zerose TM erythritol | 85.1 | - | - | 87.7 | - | - |
| Maltitol SweetPearl 300 DC | - | - | 85.1 | - | - | 87.7 |
| Sodium carbonate and. | 2.50 | 2.50 | - | - | - | 2.50 |
| Sodium bicarbonate | 4.00 | 4.00 | - | - | - | 4.00 |
| Effersoda | - | - | 6.50 | 6.50 | 6.50 | - |
| Peppermint flavor | 2.50 | 2.50 | 2.50 | - | - | - |
| Menthol | 1.00 | 1.00 | 1.00 | - | - | - |
| Eucalyptos flavor | - | - | - | 3.50 | 3.50 | 3.50 |
| Acesulfame potassium | 0.15 | 0.15 | 0.15 | 0.16 | 0.16 | 0.16 |
| Total | 100 | 100 | 100 | 100 | 100 | 100 |

When further adding magnesium stearate, this may be added by full powder mixture during the last few minutes of the final mixing.

As described below, the tablets according to the invention may be made from a wide range of different formulations.

As can be seen in table 1, microcrystalline cellulose is used as a filler. Lower amount of filler such as microcrystalline cellulose may also be used. Examples of usable fillers include magnesium- and calcium carbonate, sodium sulphate, ground limestone, silicate compounds such as magnesium- and aluminum silicate, kaolin and clay, aluminum oxide, silicium oxide, talc, titanium oxide, mono-, di- and tri-calcium phosphates, cellulose polymers, such as wood, starch polymers, fibers and combinations thereof.

As can be seen in table 1, mannitol is used as a bulk sweetener. Examples of usable bulk sweeteners include sugar sweetener and/or sugarless sweetener.

The bulk sweeteners may often support the flavor profile of the formulation.

Sugarless sweeteners generally include, but are not limited to sugar alcohols (also sometimes referred to as polyols) such as sorbitol, erythritol, xylitol, maltitol, mannitol, lactitol, and isomalt.

Sugar sweeteners generally include, but are not limited to saccharide-containing components, such as sucrose, dextrose, maltose, saccharose, lactose, sorbose, dextrin, trehalose, D-tagatose, dried invert sugar, fructose, levulose, galactose, corn syrup solids, glucose syrup, hydrogenated glucose syrup, and the like, alone or in combination. These sugar sweeteners may also be included as a humectant.

As can be seen in table 1 and 3A-3F, crospovidone, croscarmellose sodium, and sodium starch glycolate are used as disintegrants in fast disintegrating tablets. Examples of usable disintegrants include starch, pregelatinated starch, modified starch (including potato starch, maize starch, starch 1500, sodium starch glycolate and starch derivatives), cellulose, microcrystalline cellulose, alginates, ion-exchange resin, and superdisintegrants, such as crosslinked cellulose (such as sodium carboxy methyl cellulose), crosslinked polyvinyl pyrrolidone (PVP), crosslinked starch, crosslinked alginic acid, natural superdisintegrants, and calcium silicate, and combinations thereof.

As can be seen in table 1 and 3A-3G, sucralose is used as a high intensity sweetener. Usable high intensity sweeteners include, but are not limited to sucralose, aspartame, salts of acesulfame, such as acesulfame potassium, alitame, saccharin and its salts, cyclamic acid and its salts, glycyrrhizin, dihydrochalcones, thaumatin, monellin, stevioside and the like, alone or in combination.

As can be seen in table 1 and 3A-3G, peppermint and menthol are used as flavors. Usable flavors include almond, almond amaretto, apple, Bavarian cream, black cherry, black sesame seed, blueberry, brown sugar, bubblegum, butterscotch, cappuccino, caramel, caramel cappuccino, cheesecake (graham crust), cinnamon redhots, cotton candy, circus cotton candy, clove, coconut, coffee, clear coffee, double chocolate, energy cow, graham cracker, grape juice, green apple, Hawaiian punch, honey, Jamaican rum, Kentucky bourbon, kiwi, koolada, lemon, lemon lime, tobacco, maple syrup, maraschino cherry, marshmallow, menthol, milk chocolate, mocha, Mountain Dew, peanut butter, pecan, peppermint, raspberry, banana, ripe banana, root beer, RY 4, spearmint, strawberry, sweet cream, sweet tarts, sweetener, toasted almond, tobacco, tobacco blend, vanilla bean ice cream, vanilla cupcake, vanilla swirl, vanillin, waffle, Belgian waffle, watermelon, whipped cream, white chocolate, wintergreen, amaretto, banana cream, black walnut, blackberry, butter, butter rum, cherry, chocolate hazelnut, cinnamon roll, cola, creme de menthe, eggnog, English toffee, guava, lemonade, licorice, maple, mint chocolate chip, orange cream, peach, pina colada, pineapple, plum, pomegranate, pralines and cream, red licorice, salt water taffy, strawberry banana, strawberry kiwi, tropical punch, tutti frutti, vanilla, or any combination thereof.

According to an embodiment of the invention, flavor may be used as taste masking for the nicotine.

In some embodiments of the invention, the formulation comprises pH regulating agent.

In some embodiments of the invention, the formulation comprises pH regulating agent in an amount of 2.7 to 5.7% by weight of said formulation.

In some embodiments of the invention, the pH regulating agent comprises buffer.

As can be seen in table 1 and 3A-3G, sodium carbonate is used as a buffering agent. Usable buffering agents include carbonate, including monocarbonate, bicarbonate and sesquicarbonate, glycerinate, phosphate, glycerophosphate, acetate, glyconate or citrate of an alkali metal, ammonium, tris buffer, amino acids and mixtures thereof. Encapsulated buffer such as Effersoda may also be used.

In some embodiments, the formulation comprises buffering agent in an amount of from 2.7 to 5.7% by weight of the formulation .

The buffering agent may be added to the formulation together with the water-soluble fast disintegrating tablet ingredients.

When buffering agent is added to the fast disintegrating tablet as part of the water-soluble fast disintegrating tablet ingredients, a pH-profile according to embodiments of the present invention can be obtained.

Buffering agent in the tablet may be used to obtain the desired pH-values in the saliva of a tablet user.

In some embodiments, the buffering agent comprises sodium carbonate and sodium bicarbonate, e.g. in a weight-ratio between 5:1 and 2.5:1, preferably in a weight-ratio between 4.1:1 and 3.5:1.

A high suitable buffering agent according to advantageous embodiments of the present invention is the sodium carbonate - sodium bicarbonate buffer system.

As can be seen in table 1, silicon dioxide is used as a glidant. Other glidants usable for the formulation may also be used within the scope of the invention.

As can be seen in table 1 and 3A-3G, magnesium stearate is used as a lubricant. Other lubricants usable for the formulation may also be used within the scope of the invention.

As can be seen in table 3A-3F, ready to use systems may be used for preparation of tablets. Typically, such ready-to-use systems may e.g. replace filler, disintegrant, glidant or similar with a single powder mix. Suitable ready-to-use systems for the purpose, but not limited to, include Pearlitol Flash (Roquette), Pharmaburst 500 (SPI Pharma), Ludiflash (BASF), ProSolv (JRS Pharma), ProSolv EasyTab (JRS Pharma), F-Melt (Fuji Chemical), SmartEx50 or SmartEx100 (Shin Etsu / Harke Pharma).

In order to obtain a formulation designed to release the content of nicotine within a period of 90 seconds in contact with oral saliva and a formulation designed to release the content of pH regulating agent within a period of 60 seconds in contact with oral saliva a range of parameters can be adjusted.

First, by varying the composition, the disintegration time can be altered. Using ingredients with a high water-solubility may facilitate a lowered disintegration time. A lower disintegration time greatly promotes a fast release of nicotine as well as fast release of the pH regulating agent.

Particularly, including a disintegrant may significantly influence the disintegration time, subject to the total composition. Also, by varying the amount and type of the disintegrant, the disintegration time may be further adjusted. For example, if a tablet having a lower disintegration time is desired, the percentage content of disintegrant may be increased and/or the type of disintegrant may be at least partly exchanged for a more effective disintegrant.

Also, decreasing the particle size of the disintegrant tends to lower the disintegration time, likely due to an increased surface area to volume ratio.

Furthermore, the compression force used in compressed tablets correlate significantly with the obtained hardness, such that a high compression force typically increases the hardness of the obtained tablet. By adjusting the hardness of a tablet, the disintegration time may also be influenced, such that a lowered hardness typically gives a shorter disintegration time. Here it has been observed for a number of compositions that by applying the correct compression force a disintegration time below 60 second upon oral administration can be achieved, whereas a too high compression force may result in a longer disintegration time above 60 seconds. In this regard it is noted that the threshold compression force may vary significantly, depending on other parameters, such as overall composition, content and type of disintegrant, etc. When, for example, a certain setup results in a too slow disintegration, a further way of adjusting may be to replace a regular disintegrant with a superdisintegrant, i.e. which facilitates disingration in a more efficient way.

Increasing the water-solubility may also be facilitated by exchanging ingredients with low water-solubility with ingredients having higher water-solubility. For example, using sugar alcohols as fillers may be very advantageous insofar that the sugar alcohols have a higher water solubility than alternative fillers.

Moreover, when the formulation is provided as a tablet, using sugar alcohols with a lower compactibility leads to lower disintegration time. Too low compactibility may compromise the mechanical strength of the tablet and lead to undesirably high friability and risk of cracks etc.

Further examples of parameters that may be adjusted in order to obtain a formulation designed to release the content of nicotine within a period of 90 seconds in contact with oral saliva and a formulation designed to release the content of pH regulating agent within a period of 60 seconds in contact with oral saliva include size and shape of the tablet, when the formulation is provided in the form of a tablet. The larger the tablet, the longer the disintegration time and thus release time of the nicotine and pH regulating agent.

For example, in such tablet embodiments, increasing the flatness (e.g. quantified by a diameter to height ratio) for a disc-shaped tablet typically increases disintegration time by increasing the surface-to-volume. As long as the tablet has a satisfactory mechanical strength, flatness may be increased.

Also, in such tablet embodiments, modifying the cross-sectional profile from a convex type tablet to a concave shaped tablet lowers the disintegration time. It is noted that this may to some degree lower the mechanical strength of the tablet, however, as long as it is satisfactory, pursuing the concave cross-section may help to increase disintegration and thus lower the disintegration time.

Also, regardless of using tablets or powder formulations, when using binders, e.g. to obtain a higher cohesiveness and mechanical strength of the tablet or formulation, the amount of such binders may be decreased as much as possible to obtain a higher disintegration rate and thus a shorter disintegration time.

Furthermore, by adding a salivation agent to the formulation, an increased amount of saliva in the vicinity of the formulation may be facilitated, which again supports the dissolving and disintegration of the formulation to reduce the disintegration time.

A further parameter that can be adjusted is the form of nicotine used. If a faster release time of nicotine is desired, a form of nicotine that has a fast release from any carrier may be used. Even faster is using nicotine without carrier, such as free base nicotine or nicotine salt.

Further, the type and amount of lubricant, if any, may be adjusted to optimize disintegration time. For example, using Sodium stearyl fumarate (SSF) typically leads to a lower disintegration time compared to when using magnesium stearate MgSt. Typically, the formulation comprises of ingredients selected from the group consisting of bulk sweeteners, fillers, ready to use systems, flavors, dry-binders, disintegrant, hereunder superdisintegrants, tabletting aids, anti-caking agents, emulsifiers, antioxidants, enhancers, absorption enhancers, buffering agents, high intensity sweeteners, colors, glidants, lubricants, or any combination thereof. Here, tabletting aids are used for tablets but not for other powder formulations. Absorption enhancers may include e.g. pH regulating agents, such as buffering agents, and mucoadhesive.

In an embodiment of the invention, the tablet core is provided with an outer coating.

In an embodiment of the invention, said outer coating is selected from the group consisting of hard coating, soft coating and edible film-coating or any combination thereof.

According to an embodiment of the invention, at least a part of the nicotine is adhered to dry-binder particles.

According to an embodiment of the invention, an amount of dry-binder is used to adhere nicotine to bulk sweetener.

According to an embodiment of the invention, said fast disintegrating tablet comprises one or more encapsulation delivery systems.

### Example 3

### In vivo pH

The fast disintegrating tablets are designed to have an in vivo pH higher than the resting saliva pH in the mouth. Thus, pH is measured in vivo, as follows:
At least 6 individuals chewed on a gum base free of buffer for 1 minute, after which the initial pH in a sample from the saliva from each of the individuals is measured with a suitable pH-electrode system, e.g. a stainless-steel electrode PHW77-SS. Only individuals having, after chewing on a gum base free of buffer for one minute, an initial pH in the saliva inside the range from 6.7 and 7.3 are selected. These individuals thereby qualify as average individuals.

One tablet is administered sublingually to at least six individuals. Hereafter, the saliva pH from each of the six individuals is measured at specified time intervals. Thus, each pH-value is the arithmetic mean of six measurements performed on saliva-samples from six individuals.

The sample volume of the individual saliva-samples may vary because the volume of saliva obtained may be different from each individual. This difference in sample volume does not affect the pH-measurements significantly. Also, it has been established by appropriate tests that a variation in time between collections of samples does not significantly alter the result. This means that the measured pH-value after three minutes is not significantly affected by whether another saliva-sample is taken from the six individuals e.g. after two minutes or not. Furthermore, it has been established by appropriate tests that the time from taking a sample to the time of measuring is not critical to the measured value. However, in the present measurements, the pH-values were measured in the samples within at most 15 minutes of sample collection.

The results are shown in table 3J.

**Table 3J. In vivo pH. Nicorette Microtab (2 mg), Nicotinell Mint Lozenge (2 mg), and Nicotinell Mint Chewing gum (2 mg) were commercially available products.**

| | **pH** | | | |
|---|---|---|---|---|
| | **10 sec** | **20 sec** | **90 sec** | **In vivo DT (sec)** |
| FDT (12) (1 mg) | 9.3 | 9.1 | 8.4 | 20 |
| FDT (14) (1 mg) | 7.4 | 7.2 | 7.6 | 210 |
| FDT (13) (1 mg) | 5.3 | 5.8 | 6.5 | 20 |
| Pouch PPC(1) (3 mg) | 7.1 | 7.3 | 7.3 | NA |
| Nicorette Microtab (2 mg) | 6.7 | 6.8 | 6.8 | >600 |
| Nicotinell Mint Lozenge (2 mg) | 6.9 | 7.1 | 7.2 | >600 |
| Nicotinell Mint Chewing gum (2 mg) | 7.2 | 7.4 | 7.6 | NA |

As can be seen from table 3J, the pH exceeds 7.5 for FDT 12 and 13. For FDT 12, this even applies already at 10 and 20 seconds from contact with oral saliva. FDT(13), made without any buffer, did not give a pH above 7.5.

The needed raise in saliva pH is at least 0.5 - 1.0 pH units. A conventional nicotine mouth spray was chosen for comparison as well as Nicorette Microtab, Nicotinell Mint Lozenge, and Nicotinell Mint chewing gum. The conventional nicotine mouth spray reveals also fast craving relief. The conventional nicotine mouth spray raises the pH in saliva up to a maximum of 8.5 according to internal measurements. None of Nicorette Microtab and Nicotinell Mint Lozenge resulted in pH above 7.2. Nicotinell Mint chewing gum did not result in pH above 7.6.

The sample volume of the individual saliva-samples may vary because the volume of saliva obtained may be different from each individual. This difference in sample volume does not affect the pH-measurements significantly.

It should be noted that the in vivo pH would be different from an in vitro pH due to the fact that acidic sodium bicarbonate is normally continuously produced in saliva, hence neutralizing the alkaline contribution from buffer. Thus, the pH obtained in vivo will be lower than in vitro measured by e.g. dissolving the tablet in a beaker.

### Example 4

### Disintegration of nicotine tablets

The in vitro disintegration of the fast disintegrating tablets of example 1 and 2 was carried out in accordance to European Pharmacopeia 9.0, section 2.9.1, *Disintegration of tablets and capsules.* As described in the examples each batch has been manufactured in various tablet sub lots where the compression force has been varied and therefore the output parameters like hardness and friability will also vary. These output parameters do also have an impact on in vitro disintegration. The results for example 1 are outlined in table 4. A minimum and a maximum value for measured disintegration are given and this is more or less a function of the hardness.

**Table 4 - In vitro disintegration, hardness, friability. Time is given in seconds.**

| | **Mean in vitro disintegration (see)** | | **Mean hardness (N)** | | **Mean friability (%)** | |
|---|---|---|---|---|---|---|
| | **Min (sec)** | **Max (sec)** | **Min (N)** | **Max (N)** | **Min (%)** | **Max (%)** |
| FDT(a) | 21 | 24 | 14 | 63 | 0.0 | 0.3 |
| FDT(b) | 23 | 98 | 12 | 50 | 0.0 | 0.6 |
| FDT(c) | 29 | 177 | 14 | 55 | 0.0 | 0.5 |
| FDT(d) | 15 | 177 | 19 | 62 | 0.0 | 0.0 |
| FDT(e) | 13 | 175 | 15 | 45 | 0.0 | 0.2 |
| FDT(f) | 11 | 259 | 14 | 43 | 0.0 | 0.2 |

The above table should be interpreted as illustrated in the following example. When looking at e.g. FDT(a), the minimum mean disintegration time of 21 seconds correspond to a tablet pressed just hard enough to obtain a cohesive tablet having a minimum mean hardness of 14 N and a friability of 0.3%. Similarly, the maximum mean disintegration time of 24 seconds correspond to another tablet pressed harder to have a maximum mean hardness of 63 N. In this way, the tablet having a mean friability of 0.0% of FDT(a) corresponds to the tablet having a mean hardness of 63 N. In other words, in table 4 FDT(a) refers to two different tablets pressed at two different pressures, the linking being indicated above. I.e. each line corresponds to two different tablets, one for Min values of disintegration time and hardness and the Max value for friability, and another for Max values of disintegration time and hardness and the Min value for friability.

The results for example 2 are outlined in table 5.

**Table 5 - In vitro disintegration, hardness, friability. Time is given in seconds.**

| | **Mean in vitro disintegration (sec)** | | **Mean hardness (N)** | | **Mean friability (%)** | |
|---|---|---|---|---|---|---|
| | **Min (sec)** | **Max (sec)** | **Min (N)** | **Max (N)** | **Min (%)** | **Max (%)** |
| FDT(g) | 120 | 210 | 17 | 22 | N/A | 0.5 |
| FDT(h) | 40 | 80 | 16 | 24 | 0.5 | 0.8 |
| FDT(i) | 10 | 46 | 17 | 22 | 0.3 | 0.3 |
| FDT(j) | 42 | 150 | 17 | 22 | 0.7 | 1.0 |
| FDT(k) | 45 | 201 | 17 | 22 | 0.6 | 0.9 |

The above table should be interpreted as illustrated in the example below table 4.

It is seen that the in vitro disintegrating may vary a lot between the disclosed fast disintegrating tablets. Hereby a disintegration profile as desired may be used together with a high in vivo pH (as described in example 3), whereby the nicotine may be more efficiently used. Most preferable an in vitro disintegrating profile below 60 seconds is desired since it would ensure a high concentration of nicotine combined with relatively high in vivo pH.

The in vitro disintegration is a fast method to determine the time and mechanism for tablet performance. More preferable or in combination the in vivo disintegration is measured. The in vivo disintegration time is a value for the actual disintegration of the sublingual tablet under the tongue. Table 6 and 7 highlights the results for in vivo disintegration.

**Table 6 - In vivo disintegration. Time is given in seconds.**

| | **Mean in vivo disintegration (sec)** | |
|---|---|---|
| | **Min (sec)** | **Max (sec)** |
| FDT(a) | 34 | 52 |
| FDT(b) | 18 | 27 |
| FDT(c) | 37 | N/A |
| FDT(d) | 42 | N/A |
| FDT(e) | 46 | N/A |

**Table 7 - In vivo disintegration. Time is given in seconds.**

| | **Mean in vivo disintegration (sec)** | |
|---|---|---|
| | **Min (sec)** | **Max (sec)** |
| FDT(g) | 19 | 40 |
| FDT(h) | 13 | 48 |
| FDT(i) | 32 | 80 |
| FDT(j) | N/A | 56 |
| FDT(k) | N/A | 81 |

The above tables 6-7 should be interpreted as illustrated in the example below table 4.

As recognized for the in vitro disintegration results above the speed of in vivo disintegrating may be varied between the disclosed formulations. The disintegration time should be complete within 60 seconds from the onset of disintegration or preferable faster.

Since dissolution of nicotine bitartrate is a relatively fast process, the time used to release the content of nicotine can be taken as the disintegration time of the matrix (here the tablet).

### Example 5

### Nicotine release and absorption

Measurements of nicotine concentration is performed as follows:
One dose of the tablets of example 1 and 2 is administered sublingually to at least six individuals. At specified time intervals, the saliva is collected. The experiment is repeated. Thus, each nicotine concentration value is the arithmetic mean of 12 measurements, i.e. performed on saliva-samples from six individuals times 2. The nicotine concentration of saliva is analyzed on HPLC after extraction into relevant buffer.

Results are shown in tables 8A-8C.

**Table 8A. Amount of nicotine in saliva. N/A = Not applicable (not assessed)**

| | **Measuring time from initial contact with oral saliva [seconds]** | | |
|---|---|---|---|
| | **10** | **20** | **90** |
| | Concentration of nicotine [mg/mL] | | |
| 2 mg Nicotinell Mint Lozenge | 0.06 | 0.05 | 0.10 |
| 1 mg FDT (12) | 0.52 | 0.59 | 0.52 |
| 1 mg FDT (13) | 0.74 | 0.66 | 0.66 |
| 1 mg FDT (14) | 0.36 | 0.39 | 0.33 |
| 3 mg Pouch PPC 1 | 0.04 | 0.07 | 0.17 |
| Nicorette Microtab 2 mg | 0.03 | 0.05 | 0.13 |
| Nicotinell Mint Chewing gum (2 mg) | 0.02 | 0.04 | 0.18 |

**Table 8B. Amount of nicotine in residue. N/A = Not applicable (not assessed)**

| | **Measuring time from initial contact with oral saliva [seconds]** | | |
|---|---|---|---|
| | **10** | **20** | **90** |
| | Concentration of nicotine [mg/mL] | | |
| 2 mg Nicotinell Mint Lozenge | 1.90 | 1.91 | 1.85 |
| 1 mg FDT (12) | No residue | No residue | No residue |
| 1 mg FDT (13) | No residue | No residue | No residue |
| 1 mg FDT (14) | N/A | N/A | No residue |
| 3 mg Pouch PPC 1 | N/A | N/A | 1.09 |
| Nicorette Microtab 2 mg | 1.90 | 1.87 | 1.77 |
| Nicotinell Mint Chewing gum (2 mg) | 1.98 | 1.96 | 1.72 |

**Table 8C. Absorption of nicotine. N/A = Not applicable (not assessed)**

| | **Measuring time from initial contact with oral saliva [seconds]** | | |
|---|---|---|---|
| | **10** | **20** | **90** |
| | Absorption of nicotine [% by weight] | | |
| 2 mg Nicotinell Mint Lozenge | 2 | 2 | 3 |
| 1 mg FDT (12) | 48 | 41 | 48 |
| 1 mg FDT (13) | 26 | 34 | 35 |
| 1 mg FDT (14) | N/A | N/A | 67 |
| 3 mg Pouch PPC 1 | N/A | N/A | 58 |
| Nicorette Microtab (2 mg) | 4 | 4 | 5 |
| Nicotinell Mint Chewing gum (2 mg) | 0 | 0 | 5 |

As can be seen from table 8A-8C, formulations of the invention provided very high absorption, above 40% or even above 50%. Also, since FDT 1 and 2 are comparable, only that FDT 2 does not contain buffer, the effect of inclusion of the buffer may be observed. It is noted that FDT1 has a final absorption being significantly higher than FDT2, illustrating how inclusion of buffer increases the absorption of nicotine. Also, it is observed that the absorption of nicotine is more or less constant at times 10 seconds, 20 seconds, and 90 seconds, illustrating how the disintegration time (about 10 seconds for FDT 1) is the limiting factor, and that the time for release of nicotine after disintegration as well as the time for absorption of nicotine is negligible for the present compositions.

The tablets of the example 1 and 2 are highly suitable to obtain solid oral nicotine formulations for fast onset nicotine craving relief comprising nicotine and a pH regulating agent, wherein the formulation is designed to release the content of nicotine within a period of 90 seconds in contact with oral saliva and the formulation is designed to release the content of pH regulating agent within a period of 60 seconds in contact with oral saliva.

### Example 6

### Evaluation of fast disintegrating tablets - burning

In general experiments have disclosed that nicotine fast disintegrating tablets according to the invention result in high absorption efficiency of nicotine into the blood stream for a fast disintegrating tablet user. With such fast integration, high pH-value combined with high nicotine concentration, only a minor part of the nicotine is swallowed by the user instead of entering the blood system, thereby resulting in fast craving relief.

When pH in the mouth is high, the nicotine is used in a very efficient way. However, too high pH in the saliva of the fast disintegrating tablet users may not be desirable, since the highly alkaline pH-value results in problems with irritation and burning of the sublingual tissue.

Consequently, the fast disintegrating tablets of the invention are indeed suitable in that they provide an efficient utilization of nicotine and at the same time are pleasant to the user, i.e. with clearly diminished unwanted side effects, hereunder particularly so called nicotine burning in the throat.

Evaluation of burning sensation of the throat is performed as described in the following.

Nicotine burning was evaluated by a test panel of 7 trained assessors. After calibration by means of chewing two standard nicotine containing chewing gum with "known" burning intensity, each assessor evaluates the burning sensation in the throat on a scale from 1 to 15, where 15 is the most intense burning. Each assessor evaluates all samples twice. The evaluations are noted for the time periods indicated. Average values are calculated.

**Table 9. Sensory evaluation of throat burning**

| | **Time [seconds]** | | |
|---|---|---|---|
| | 145 | 295 | 505 |
| | Burning score (1-15) | | |
| FDT (12) | 3.5 | 1.8 | 0.8 |
| FDT (14) | 6.6 | 4.5 | 2.7 |
| Nicotinell Mint Chewing gum (2 mg) | 4.6 | 4.6 | 3.4 |
| Nicotinell Mint Lozenge (2 mg) | 4.8 | 4.9 | 4.2 |
| Nicorette Microtab (2 mg) | 6.4 | 5.9 | 5.5 |

### Example 7 - Alleviation of nicotine craving

Nicotine craving alleviation was tested using a panel of three users evaluating all samples twice. Each user noted the time from oral administration until craving relief, i.e. feeling the effect of nicotine reaching the head. The average times for FDT (12) and FDT (14) and three commercially available products are indicated in table 10.

**Table 10. Time before alleviation.**

| **Time before alleviation** | **FDT (12)** | **FDT (14)** | **Nicotinell Mint Chewing gum (2 mg)** | **Nicotinell Mint Lozenge (2 mg)** | **Nicorette Microtab (2 mg)** |
|---|---|---|---|---|---|
| Average | 240 | 300 | 560 | 480 | 400 |

As can be seen from table 10, significantly faster alleviation was obtained compared to the commercially available products.

## Claims

1. An orally disintegrating nicotine tablet for fast onset nicotine craving relief comprising a pressed powder formulation, the formulation comprising nicotine and a pH regulating agent, wherein the orally disintegrating nicotine tablet is designed to release the content of nicotine within a period of 60 seconds in contact with oral saliva and the orally disintegrating nicotine tablet is designed to release the content of pH regulating agent within a period of 60 seconds in contact with oral saliva,
wherein the orally disintegrating nicotine tablet comprises at least one polyol, wherein the polyol comprises more than 40% by weight of the orally disintegrating nicotine tablet,
wherein the at least one polyol is selected from the list consisting of sorbitol, erythritol, xylitol, maltitol, mannitol, lactitol, and isomalt,
wherein the orally disintegrating nicotine tablet comprises nicotine in an amount of at least 0.5 mg, wherein the nicotine is provided as free base nicotine or nicotine salt, wherein the pH regulating agent is an alkaline buffering agent, and
wherein the nicotine is without carrier.

2. The orally disintegrating nicotine tablet according to claim 1, wherein the orally disintegrating nicotine tablet comprises said pH regulating agent in an amount of at least 2.7 % of the orally disintegrating nicotine tablet.

3. The orally disintegrating nicotine tablet according to any of claims 1-2, wherein said nicotine is provided as nicotine salt.

4. The orally disintegrating nicotine tablet according to claim 3, wherein said nicotine salt comprises nicotine bitartrate.

5. The orally disintegrating nicotine tablet according to any of claims 1-2, wherein said nicotine is provided as free nicotine base.

6. The orally disintegrating nicotine tablet according to any of claims 1-5, wherein said orally disintegrating nicotine tablet further comprises a disintegrant.

7. The orally disintegrating nicotine tablet according to any of claims 1-6, wherein the orally disintegrating nicotine tablet comprises disintegrant in an amount of 1-10 % by weight of the orally disintegrating nicotine tablet.

8. The orally disintegrating nicotine tablet according to claim 6 or 7, wherein the disintegrant is selected from starch, pregelatinated starch, modified starch (including potato starch, maize starch, starch 1500, sodium starch glycolate and starch derivatives), cellulose, microcrystalline cellulose, alginates, ion-exchange resin, and superdisintegrants, such as crosslinked cellulose (such as sodium carboxy methyl cellulose), crosslinked polyvinyl pyrrolidone (PVP), crosslinked starch, crosslinked alginic acid, natural superdisintegrants, and calcium silicate, and combinations thereof.

9. The orally disintegrating nicotine tablet according to any of claims 1-8, wherein the orally disintegrating nicotine tablet is provided as a tablet having a weight of 25 to 200mg, such as 50 to 150 mg, such as 70-120mg, such as about 75 mg or about 100 mg.

10. The orally disintegrating nicotine tablet according to any of claims 1-9, wherein the orally disintegrating nicotine tablet comprises sodium stearyl fumarate (SSF) as a lubricant.

11. The orally disintegrating nicotine tablet according to any of claims 1-10, wherein the orally disintegrating nicotine tablet comprises microcrystalline cellulose in an amount of 1-10 % by weight of the orally disintegrating nicotine tablet.

12. The orally disintegrating nicotine tablet according to any of claims 1-11, wherein the orally disintegrating nicotine tablet comprises mannitol as a bulk sweetener.

13. The orally disintegrating nicotine tablet according to any one of claim 1 to 12 for use in alleviation of nicotine craving.

## Patentansprüche

1. Im Mund zerfallende Nikotintablette für einen schnellen Beginn einer Linderung eines Verlangens nach Nikotin, umfassend eine gepresste Pulverformulierung, wobei die Formulierung Nikotin und ein pH-regulierendes Mittel umfasst, wobei die im Mund zerfallende Nikotintablette konzipiert ist, um den Nikotingehalt innerhalb eines Zeitraums von 60 Sekunden bei Kontakt mit oralem Speichel freizugeben, und die im Mund zerfallende Nikotintablette konzipiert ist, um den Gehalt des pH-regulierenden Mittels innerhalb eines Zeitraums von 60 Sekunden bei Kontakt mit oralem Speichel freizugeben,
wobei die im Mund zerfallende Nikotintablette mindestens ein Polyol umfasst, wobei das Polyol mehr als 40 Gew.-% der im Mund zerfallenden Nikotintablette ausmacht,
wobei das mindestens eine Polyol aus der Liste ausgewählt ist, bestehend aus Sorbit, Erythrit, Xylit, Maltit, Mannit, Lactit und Isomalt,
wobei die im Mund zerfallende Nikotintablette Nikotin in einer Menge von mindestens 0,5 mg umfasst, wobei das Nikotin als freie Nikotinbase oder Nikotinsalz bereitgestellt ist, wobei das pH-regulierende Mittel ein alkalisches Puffermittel ist und wobei das Nikotin ohne Träger ist.

2. Im Mund zerfallende Nikotintablette nach Anspruch 1, wobei die im Mund zerfallende Nikotintablette das pH-regulierende Mittel in einer Menge von mindestens 2,7 % der im Mund zerfallenden Nikotintablette umfasst.

3. Im Mund zerfallende Nikotintablette nach einem der Ansprüche 1 bis 2, wobei das Nikotin als Nikotinsalz bereitgestellt wird.

4. Im Mund zerfallende Nikotintablette nach Anspruch 3, wobei das Nikotinsalz Nikotinbitartrat umfasst.

5. Im Mund zerfallende Nikotintablette nach einem der Ansprüche 1 bis 2, wobei das Nikotin als freie Nikotinbase bereitgestellt wird.

6. Im Mund zerfallende Nikotintablette nach einem der Ansprüche 1 bis 5, wobei die im Mund zerfallende Nikotintablette ferner ein Sprengmittel umfasst.

7. Im Mund zerfallende Nikotintablette nach einem der Ansprüche 1 bis 6, wobei die im Mund zerfallende Nikotintablette das Sprengmittel in einer Menge von 1 bis 10 Gew.-% der im Mund zerfallenden Nikotintablette umfasst.

8. Im Mund zerfallende Nikotintablette nach Anspruch 6 oder 7, wobei das Sprengmittel aus Stärke, vorverkleisterter Stärke, modifizierter Stärke (einschließlich Kartoffelstärke, Maisstärke, Stärke 1500, Natriumstärkeglykolat und Stärkederivaten), Zellulose, mikrokristalliner Zellulose, Alginaten, Ionenaustauscherharz und Supersprengmitteln wie vernetzter Zellulose (wie Natriumcarboxymethylzellulose), vernetztem Polyvinylpyrrolidon (PVP), vernetzter Stärke, vernetzter Alginsäure, natürlichen Supersprengmitteln und Calciumsilikat und Kombinationen davon ausgewählt ist.

9. Im Mund zerfallende Nikotintablette nach einem der Ansprüche 1 bis 8, wobei die im Mund zerfallende Nikotintablette als eine Tablette, die ein Gewicht von 25 bis 200 mg, wie 50 bis 150 mg, wie 70 bis 120 mg, wie etwa 75 mg oder etwa 100 mg, bereitgestellt wird.

10. Im Mund zerfallende Nikotintablette nach einem der Ansprüche 1 bis 9, wobei die im Mund zerfallende Nikotintablette Natriumstearylfumarat (SSF) als ein Gleitmittel umfasst.

11. Im Mund zerfallende Nikotintablette nach einem der Ansprüche 1 bis 10, wobei die im Mund zerfallende Nikotintablette mikrokristalline Cellulose in einer Menge von 1 bis 10 Gew.-% der im Mund zerfallenden Nikotintablette umfasst.

12. Im Mund zerfallende Nikotintablette nach einem der Ansprüche 1 bis 11, wobei die im Mund zerfallende Nikotintablette Mannit als einen Zuckeraustauschstoff umfasst.

13. Im Mund zerfallende Nikotintablette nach einem der Ansprüche 1 bis 12 zur Verwendung bei der Linderung des Verlangens nach Nikotin.

## Revendications

1. Comprimé de nicotine à désintégration orale destiné au soulagement rapide du manque de nicotine, comprenant une formulation de poudre pressée, la formulation comprenant de la nicotine et un agent régulateur de pH, dans lequel le comprimé de nicotine à désintégration orale est conçu pour libérer le contenu de nicotine dans un délai de 60 secondes en contact avec la salive orale et le comprimé de nicotine à désintégration orale est conçu pour libérer le contenu de l'agent régulateur de pH dans un délai de 60 secondes en contact avec la salive orale,
dans lequel le comprimé de nicotine à désintégration orale comprend au moins un polyol, dans lequel le polyol comprend plus de 40 % en poids du comprimé de nicotine à désintégration orale,
dans lequel l'au moins un polyol est choisi parmi la liste constituée du sorbitol, de l'érythritol, du xylitol, du maltitol, du mannitol, du lactitol et de l'isomalt,
dans lequel le comprimé de nicotine à désintégration orale comprend de la nicotine en une quantité d'au moins 0,5 mg, dans lequel la nicotine est fournie sous forme de nicotine de base libre ou de sel de nicotine, dans lequel l'agent régulateur de pH est un agent tampon alcalin, et dans lequel la nicotine est dépourvue du support.

2. Comprimé de nicotine à désintégration orale selon la revendication 1, dans lequel le comprimé de nicotine à désintégration orale comprend ledit agent régulateur de pH en une quantité d'au moins 2,7 % du comprimé de nicotine à désintégration orale.

3. Comprimé de nicotine à désintégration orale selon l'une quelconque des revendications 1 à 2, dans lequel ladite nicotine est fournie sous forme de sel de nicotine.

4. Comprimé de nicotine à désintégration orale selon la revendication 3, dans lequel ledit sel de nicotine comprend du bitartrate de nicotine.

5. Comprimé de nicotine à désintégration orale selon l'une quelconque des revendications 1 à 2, dans lequel la nicotine est fournie sous forme de nicotine à base libre.

6. Comprimé de nicotine à désintégration orale selon l'une quelconque des revendications 1 à 5, dans lequel ledit comprimé de nicotine à désintégration orale comprend en outre un désintégrant.

7. Comprimé de nicotine à désintégration orale selon l'une quelconque des revendications 1 à 6, dans lequel le comprimé de nicotine à désintégration orale comprend un désintégrant en une quantité de 1 à 10 % en poids du comprimé de nicotine à désintégration orale.

8. Comprimé de nicotine à désintégration orale selon la revendication 6 ou 7, dans lequel le désintégrant est choisi parmi l'amidon, l'amidon prégélatinisé, l'amidon modifié (y compris l'amidon de pomme de terre, l'amidon de maïs, l'amidon 1500, le glycolate d'amidon sodique et les dérivés de l'amidon), la cellulose, la cellulose microcristalline, les alginates, la résine échangeuse d'ions et les superdésintégrants, tels que la cellulose réticulée (comme la carboxyméthyl cellulose de sodium), la polyvinylpyrrolidone (PVP) réticulée, l'amidon réticulé, l'acide alginique réticulé, les superdésintégrants naturels et le silicate de calcium, et des combinaisons de ceux-ci.

9. Comprimé de nicotine à désintégration orale selon l'une quelconque des revendications 1 à 8, dans lequel le comprimé de nicotine à désintégration orale est fourni sous la forme d'un comprimé ayant un poids de 25 à 200 mg, tels que 50 à 150 mg, tels que 70 à 120 mg, tels qu'environ 75 mg ou environ 100 mg.

10. Comprimé de nicotine à désintégration orale selon l'une quelconque des revendications 1 à 9, dans lequel le comprimé de nicotine à désintégration orale comprend du fumarate de stéaryle de sodium (SSF) comme lubrifiant.

11. Comprimé de nicotine à désintégration orale selon l'une quelconque des revendications 1 à 10, dans lequel le comprimé de nicotine à désintégration orale comprend de la cellulose microcristalline en une quantité de 1 à 10 % en poids du comprimé de nicotine à désintégration orale.

12. Comprimé de nicotine à désintégration orale selon l'une quelconque des revendications 1 à 11, dans lequel le comprimé de nicotine à désintégration orale comprend du mannitol en tant qu'édulcorant en vrac.

13. Comprimé de nicotine à désintégration orale selon l'une quelconque des revendications 1 à 12 destiné à l'atténuation de l'envie de nicotine.
